# NEUE EUROPÄISCHE PATENTSCHRIFT

(11) **EP 0 530 173 B2**
(45) Veröffentlichungstag und Bekanntmachung des Hinweises auf die Entscheidung über den Einspruch: **22.08.2001**
(45) Hinweis auf die Patenterteilung: 05.03.1997
(21) Anmeldenummer: 92890128.9
(22) Anmeldetag: 26.05.1992
(51) Int. Cl.: A61L 2/00, A61L 2/18

(54) **Verfahren zur Inaktivierung von Prionen**
Method for inactivating prions
Procédé pour l'inactivation de prions

(30) Priorität: 19.08.1991 AT 162991
(43) Veröffentlichungstag der Anmeldung: 03.03.1993
(73) Patentinhaber: HÄMOSAN ERZEUGUNG PHARMAZEUTISCHER GRUNDSTOFFE GmbH, A-8262 Ilz (AT)
(72) Erfinder: Reichl, Herwig, A-8020 Graz (AT)
(74) Vertreter: Müllner, Erwin, Dr.

(56) Entgegenhaltungen:
- PATENT ABSTRACTS OF JAPAN, vol. 12, no. 444 (C-545), 22. November 1988; & JP-A-63 166 835
- Biochemistry I, John Wiley & Sons, Inc., 1990, Seite 181
- Methods in Enzymplogy, Bd. 182, 1980, Seite 290
- Römpp Chemie Lexikon, 1989, Seite 631
- Proc. Natl. Acad. Sci., Bd. 78(7), 1981, S. 4606-4610

## Beschreibung

Die vorliegende Erfindung betrifft ein Verfahren zur Inaktivierung von Prionen und Viren in biologischen oder biogenen Materialien, insbesondere in einem als Ausgangsmaterial für Albumin geeigneten Plasma oder Serum, unter Verwendung eines chaotropen Reagens.

Durch das Verfahren gemäß der vorliegenden Erfindung sollen also bei der Darstellung von Proteinen, insbesondere Serum-albuminen, eventuell vorhandene Prionen u. Viren zuverlässig inaktiviert werden.

Bei der Herstellung von Proteinen aus tierischem (oder auch menschlichem) Material ist immer die Gefahr der Verunreinigung mit infektiösen Partikeln gegeben, welche im Ausgangsmaterial zugegen waren. Ein tragisches Beispiel der jüngsten Vergangenheit stellt die Infektion vieler Menschen mit HIV-Viren, den Erregern von AIDS, dar, welche durch die Verabreichung von Blutderivaten an z. B. Bluter erfolgte. Dies geschah vor allem vor der Entdeckung des AIDS-Erregers; seither erfolgt die Überwachung der Blutspender, das sogenannte "screening", und außerdem suchen die Hersteller Verfahren, die eventuell trotzdem im Spenderblut auftretende HIV-Viren während der Reinigung der Serumproteine und der Endkonfektionierung der Therapeutika eliminieren.

Je mehr über die Natur des Erregers einer Krankheit bekannt ist, desto leichter und schneller können Verfahren zu seiner Zerstörung entwickelt werden. Für die (historisch gesehen) ersten infektiösen Partikel, die Bakterien, gibt es daher seit Jahrzehnten anerkannte Verfahren, die allesamt zum Stand der Technik gehören (Dampfsterilisation, Trockensterilisation, Pasteurisieren, Steril-Filtration, Ethylenoxid, Strahlungsinaktivierung, etc.).

Für die Viren, eine weitere Erregergruppe, die erst im 20. Jahrhundert entdeckt wurde, gibt es ebenfalls Verfahren zur Eliminierung, doch sind diese spezifischer auf die Art des Erregers abzustimmen. Während für manchen Viren eine pH-Wert-Absenkung auf 4 zur vollständigen Zerstörung genügt, werden andere Arten erst durch organische Lösungsmittel in hoher Konzentration inaktiviert.

Seit einigen Jahren erscheint nun eine neue Gruppe von Krankheitserregern, die sogar das zentrale Dogma der Molekularbiologie in Frage zu stellen scheint, zunehmend in wissenschaftlichen Publikationen: die Prionen. manchmal auch als unkonventionelle Viren oder "slow virus" bezeichnet. Über ihre stoffliche Natur herrscht nocht Uneinigkeit: während ein Teil der Wissenschaftler sie für extrem kleine Viren, d.h. ein kleines Stück Nukleinsäure mit einigen Hüllproteinen, hält, zieht ein zunehmend größerer Teil aus dem Fehlen des Nachweises irgendeiner Nukleinsäure sowie aus vielen anderen Befunden den revolutionären Schluß, daß es sich hiebei um infektiöses Protein ohne DNA oder RNA handelt. Dies wäre der erste Widerspruch zum Dogma, daß zur Vervielfältigung von infektiösen Partikeln Nukleinsäuren (genetisches Material) notwendig sind.

Herrscht über die Natur der Prionen noch Unklarheit, so sind doch einige Krankheiten bekannt, die Menschen oder Tiere befallen und gegen die es keine Behandlungsmöglichkeiten gibt, die also immer tödlich enden. Da es sich hiebei um Infektionskrankheiten handelt, deren Inkubationszeit bis zu 30 Jahre beträgt, ist das Unbehagen bei Forschern und Herstellern von Therapeutika sehr hoch, aber auch zunehmend beim Konsumenten: Eine dieser Krankheiten ist nämlich BSE (Bovine Spongiphorme Encephalopathien), die rätselhafte englische Rinderseuche. Eine weitere, sehr ähnliche Krankheit ist SCRAPIE bei Schaf und Ziege (vielleicht der Ursprung der jetzigen BSE-Epidemie).

Bei Menschen sind es KURU, eine Krankheit, die bei rituellen Kannibalen in Papua-Neuguinea auftritt, das JakobCreutzfeldt Syndrom sowie das Gerstmann-Sträussler Syndrom. Es bestehen jedoch auffallende Ähnlichkeiten mit der Alzheimer' schen Krankheit, und damit wäre der Kreis der exotischen Krankheiten (mit Auftrittswahrscheinlichkeiten von 1:1 Million) verlassen.

Es gibt Hinweise, daß BSE durch die Verfütterung von Tiermehl aus Tierkörperverwertungsanstalten, die auch an Scrapie leidende Schafe entsorgten, in England epidemisch wurde. Leider gibt es auch eine Entsprechung beim Menschen: bei der Herstellung von menschlichem Wachstumshormon aus menschlichen Hypophysen (aus Obduktionsmaterial) gelangten Erreger des Creutzfeldt-Jakob Syndroms in eine Charge eines Herstellers; mehrere Fälle dieser Krankheit sind bereits bei Empfängern dieser Hormoncharge aufgetreten, und zwar bei Jugendlichen, wiewohl sie sonst bei Menschen über 50 auftritt.

Dies möge genügen, die potentielle Gefährdung aufzuzeigen, die aus diesem neuen Formenkreis von Krankheiten kommt. Erschwert wird die Beurteilung der Gefährdung ebenso wie die Behandlung durch die geringe Kenntnis dieser neuen Erreger, der Prionen: es scheint, daß sie wenig oder keine Nukleinsäure besitzen und daß ihr Prion-Protein von einem Wirtsgen kodiert und später pathogen verändert wird. Außerdem verursachen Prionen keine Immunantwort wie Antikörperbildung, was die Diagnostik sehr erschwert.

Versucht man nun, Prionen zu zerstören, so ergibt sich ein weiteres Problem: sie sind extrem widerstandsfähig gegen alle physikalischen und chemischen Methoden; bisher ist nur bekannt, daß sie durch Behandlung mit konzentrierten Mineralsäuren oder Laugen, am besten bei hoher Temperatur,durch Behandlung mit Hypochlorit-Lauge oder durch Temperaturen von über 150°C eliminiert werden; es ist klar, daß derart extreme Bedingungen jedes biologische Therapeutikum sofort zerstören oder zumindest unwirksam machen.

Bisher wurde versucht, das Ausgangsmaterial aus Gegenden zu beziehen, in denen die oben erwähnten Seuchen bisher nicht aufgetreten sind, um prionfreies biologisches Material herzustellen. Bedenkt man jedoch die extrem lange Inkubationszeit von bis zu 30 Jahren, scheint diese Vorgangsweise sehr gefährlich. Durch das Screening des Ausgangsmaterials lassen sich Prionen nicht zuverlässig feststellen, weil - wie bereits erwähnt - keine Immunreaktion auftritt. Da der biologische Test (Injektion des fraglichen Materials in die Gehirne von Mäusen, Warten auf pathologisches Verhalten, Tod der Tiere, Bestätigung durch molekularbiologische und histologische Befunde sowie durch neuerliche Injektion in Mäusegerhirne) ca. 14 Monate dauert und extrem aufwendig ist, eignet er sich weder für die Kontrolle des Ausgangsmaterials noch für die Chargenüberprüfung fertiger Arzneimittel, er kann nur zur Validierung neuer Herstellungsverfahren (nach Zusatz von Prionen) dienen. Dies muß unter größten Sicherheitsvorkehrungen in speziellen Labors geschehen, in denen auch die Versuchstiere gehalten und untersucht werden (P 3-Zonen).
Es ist daher Aufgabe der vorliegenden Erfindung, ein Verfahren zu schaffen, das eventuell vorhandene Prionen in biologischem oder biogenem Material zuverlässig eliminiert, ohne dieses Material zu zerstören, und das auch alle bekannten Gruppen von Viren zerstört.

Diese Aufgabe wird durch ein Verfahren der eingangs genannten Art erfindungsgemäß dadurch gelöst, daß man das biologische oder biogene Material - gegebenenfalls nach Verdünnung mit pyrogenfreiem, sterilem Wasser - auf einen pH-Wert von etwa 6,5 einstellt und etwa 1 g/l eines Detergens, insbesondere eines anionischen Detergens, zusetzt und danach dieses Material unter Rühren langsam auf etwa 70°C erhitzt und bei dieser Temperatur mindestens 15, vorzugsweise etwa 30 min weiter rührt, wonach man es rasch abkühlt, auf einen pH von 4 bis 4,2 ansäuert und die ausgefällten Globuline abtrennt, und daß man es nach der Detergens-/Wärmebehandlung mit einem chaotropen Reagens mindestens 12, vorzugsweise 18 Stunden behandelt.

Es wurde nämlich im Rahmen der vorliegenden Erfindung überraschend gefunden, daß sich Prionen durch mäßiges Erhitzen zusammen mit einer längeren Behandlung mit einem chaotropen Reagens letzteres bei normaler Raumtemperatur (20 - 25°C) eliminieren lassen. Durch eine derartige Behandlung werden biologische Materialien wie z.B. Albumine nicht beeinträchtigt. Auch Viren und Bakterien werden dadurch zuverlässig eliminiert.

Als chaotropes Reagens wird vorzugsweise Harnstoff in einer Konzentration von 6 - 8 molar, bezogen auf die Gesamtmenge, oder Natriumthiocyanat in einer Konzentration von 1 - 2 molar, bezogen auf die Gesamtmenge, eingesetzt. Es kann nach der Behandlung durch Dialyse, Diafiltration oder Gelpermeationschromatographie entfernt werden.

Als Detergens kann ein Alkylsulfat oder ein Derivat davon, vorzugsweise Natriumlaurylsulfat, und/oder ein Sarcosinat, vorzugsweise Natrium-(N-lauroylsarcosinat) und/oder ein Alkylsulfonat oder ein Derivat davon eingesetzt werden. Den pH-Wert stellt man zweckmäßigerweise durch Zugabe verdünnter Salzsäure ein.

Vorzugsweise setzt man zusätzlich zu dem Detergens 8 - 10 Vol.-% Methyl- oder Ethylalkohol zu.

Im Rahmen der vorliegenden Erfindung wird Rinderserumalbumin (BSA) als Beispiel gewählt, weil es in großen Mengen verwendet wird, die Rohstoffquelle "Rind" durch BSE gefährdet ist und weil außerdem das menschliche Analoge, Humanes Serum Albumin (HSA), ein sehr wichtiges Therapeutikum ist. Das Verfahren läßt sich direkt ohne Abänderung auf die HSA-Gewinnung übertragen; auf andere Proteine tierischen oder menschlichen Ursprungs kann es modifiziert werden.

Das erfindungsgemäße Verfahren - angewendet auf BSA - kann wie folgt zusammengefaßt werden:

Das Plasma oder Serum, aus dem BSA oder HSA gewonnen wird, wird nach Verdünnung mit Wasser, Änderung des pH-Wertes auf 6,5 und Zusatz von Ethyl- oder Methylalkohol bei einer Temperatur von 70°C 30 min lang in Gegenwart eines starken Tensides, vorzugsweise Natriumlaurylsulfat (Dodecylsulfat Natriumsalz) oder eines Analogen, Natrium-(N-lauroylsarcosinat) oder von anderen Analogen gerührt. Danach wird rasch abgekühlt, der pH auf 4,0 bis 4,2 eingestellt, die gefällten Globuline werden entfernt und die klare Lösung wird neutralisiert. Danach wird ein stark chaotropes Reagens in hoher Konzentration zugegeben, vorzugsweise 6 - 8 molarer Harnstoff oder 1 - 2 molares Natriumthiocyanat, die Lösung 16 Stunden bei Raumtemperatur belassen und danach das Reagens entfernt, vorzugsweise durch Chromatographie, aber auch Dialyse oder Diafiltration sind möglich. Die entsalzte Lösung wird dann aufkonzentriert, eventuell mit Zusätzen versehen, und dann entweder sterilfiltriert und steril abgefüllt oder getrocknet, insbesondere durch Gefriertrocknung. Eine thermische Nachbehandlung der Lösung ("Pasteurisieren" in Gegenwart von Stabilisatoren) oder des Pulvers (Dampfinjektion) kann angeschlossen werden.

Statt Serum oder Plasma kann jedes andere Ausgangsmaterial, in dem sich Albumin befindet, verwendet werden, etwa Placentalblut, Fraktionen der Plasmafraktionierung nach Cohn, etc.

Die Wirksamkeit wurde im biologischen Modell durch Zugabe von Scrapie-Prionen zum Ausgangsplasma und Injektion der Albuminlösung in die Gehirne von Mäusen nachgewiesen, sowie durch die Zugabe hoher KOnzentrationen konventioneller Viren wie Bovine Viral Diarrhoea (BVD), Infektiöse Bovine Rhinotracheitis (IBR), Parainfluenza 3, Maul- und Klauenseuche, Maedi-Visna Virus und Parapox Virus des Schafes (ORF). Die so behandelten Albuminlösungen waren autosteril, was die Zerstörung von Bakterien beweist.

Im einzelnen wurde dabei wie folgt vorgegangen: 3 g getrocknetes Rinderplasma (entsprechend 2 g Protein) wurden in 35 ml destilliertem, sterilem Wasser gelöst. 4 ml absolutiertes Ethanol wurden zugegeben. Der pH-Wert wurde mit verdünnter Salzsäure auf 6,5 eingestellt. 45 mg (= 0,1 %) Natriumlaurylsulfat (mindestens 95 % rein) wurden aufgelöst. Insgesamt ergaben sich 44 ml Gesamtvolumen.

Unter den notwendigen Sicherheitsvorkehrungen (P 3-Labor, Laminar Flow Hood ...) wurden 225 µl eines 20%igen Scrapie-Hirn-Homogenisates (Titer: 2.10⁹ LD₅₀/g) zugegeben und homogenisiert, oder es wurden Viren zugegeben, deren Art und Konzentration in Tab. 1 angegeben sind. Die Lösung wurde in ein Wasserbad mit 70°C gestellt; nach 10 min hatte der Inhalt ebenfalls 70°C, daraufhin wurde die Behandlung noch 30 min fortgesetzt. Nach jeweils 10 min wurde 1 min lang mittels Magnetrührer geruhrt. Nach Ende der Inkubationszeit wurde die Probe auf Eis gestellt und abgekühlt. Die kalte Lösung wurde mit 200 µl Salzsäure auf pH 4,2 gebracht und nochmals homogenisiert.

Die Entfernung der gefällten Globuline erfolgte bei 6000 Umdrehungen pro Minute (entspricht 4000 g) bei 4°C während 10 min. Der klare Überstand wurde durch Abdekantieren gewonnen; er war reine Rinderalbumin-Lösung, Volumen: 20,5 ml.

Diese Lösung wurde durch Zugabe von 250 µl einer Natronlaugelösung neutralisiert (pH 7,0). Danach wurden 15 g Harnstoff zugegeben, wodurch sich das Volumen auf 30 ml vergrößerte, sodaß sich eine Konzentration von 8 molar ergab. Diese Lösung wurde 16 Stunden bei Raumtemperatur (21°C) belassen.

Zur Entfernung des Harnstoffes wurde das Verfahren der Gelpermeations-Chromatographie verwendet:
15 g Sephadex® G 50 (Fa. Pharmacia, Uppsala) wurden in 500 ml sterilem destilliertem Wasser erhitzt und über Nacht stehengelassen. Das gequollene Gel würde in eine Acrylglas-Säule mit 5 cm Durchmesser und 30 cm Höhe gefüllt und mit sterilem destilliertem Wasser gewaschen (200 ml). Die Flußrate war 7,5 ml/min.
30 ml der obigen Lösung wurden aufgetragen, danach wurde mit 100 ml Wasser nachgewaschen; die Fraktionen wurden wie folgt vereinigt: 50 + 40 ml. Danach war kein Protein mehr im Eluat feststellbar. Die 90 ml wurden lyophilisiert und ergaben 0,7 g Rinderserum-Albumin.

Alternativ wurde der Harnstoff in einigen Experimenten mittels Diafiltration in einem Amicon® S-1 Modul, cut-off 10.000 d. entfernt. Dabei wurde mit etwa 1000 ml dest. Wasser diafiltriert und anschließend auf ca. 50 ml aufkonzentriert, danach lyophilisiert. Biologische Testung auf Scrapie/BSE:

Dieses Albumin wurde in 3 ml physiologischer Kochsalzlösung gelöst und in aliquoten Mengen von 20 µl Mäusen in jeweils eine Hirnhälfte injiziert. Gesamtzahl der Mäuse: 136. Dieverwendeten Mäuse waren vom Stamm C57/B16.

Als Positivkontrolle diente eine Verdünnungsreihe des Scrapie-Inoculums (s. o.,2.10⁹ LD₅₀/g), 8 Verdünnungsstufen. Als Negativkontrolle diente Rinderserumalbumin, das wie oben, jedoch ohne Scrapie-Inoculum hergestellt worden war.

Alle Inokulationen erfolgten an jeweils 12 Tieren, d. h. in 12facher Wiederholung.

Als Ergebnis zeigte sich, daß der Titer in der Positivkontrolle bestätigt werden konnte, während weder in der Negativkontrolle noch in der Versuchsgruppe das Krankheitsbild oder Todesfälle auftraten.

Biologische Testung im Fall der konventionellen Viren:

Das BSA wurde in Zellkulturmedium aufgelöst. Dieses Medium diente als Nährmedium für Säugetierzellen, welche für das jeweilige Virus als Wirtszellen dienen können (siehe Tabelle 1). Positive (Virusstammlösung) und negative (nur Medium) Versuche sowie Tests auf unspezifische Cytopathogenität von Albumin vervollständigten diese Tests.

Die Abtötungsraten sind in Tabelle 1 angegeben und waren in allen Fällen durch die Ausgangstiter begrenzt.

**Tabelle 1**

| Virus | Ausgangskonz. TC1D | Wirtszelle | überleb. Viren | Abtötrate |
|---|---|---|---|---|
| BVD Stamm "Singer", pass.9 | 1x10⁸ | | 0 | 2,5x10⁶ |
| IBR Stamm "ames", pass.18 | 1,8x10⁶ | | 0 | 7 x 10⁴ |
| PI 3 Stamm "Freistadt", pass.78 | 80 HTH units | | 0 | -- |
| MKS O 1 BFS 1860, pass.5 | 2 x 10⁷ | | 0 | 1 x 10⁶ |
| MVV (ATCC-VR-779) | 1.2x10⁹ | WSCP | 0 | 2,9x10⁶ |
| ORF (Dept.of Path., Univ. Glasgow) | 2x10⁹ | PAL-6 | 0 | 3,4x10⁶ |

Die Äquivalenz von N-Lauroylsarcosinat mit Laurylsulfat als Detergens bei der Darstellung von Albumin aus Plasma wurde in einem ähnlichen Versuch, jedoch ohne Zugabe von infektiösem Material gezeigt; die gemessenen Parameter waren Ausbeute und Proteinreinheit (bei beiden Detergentien über 98 %).

Die Äquivalenz von Harnstoff und Natriumthiocyanat als chaotrope Reagenzien wurde bei der Behandlung von Ribonukleoprotein-Partikeln von Influenzaviren (einem Modell für Nukleinsäure-Protein-Wechselwirkungen) und bei der Aufhebung von Affinitätsbindungen, wie z. B. bei Gelatin-Fibronectin, erprobt und nachgewiesen.

## Patentansprüche

1. Verfahren zur Inaktivierung von Prionen und Viren in biologischen oder biogenen Materialien, insbesondere in einem als Ausgangsmaterial für Albumin geeigneten Plasma oder Serum, unter Verwendung eines chaotropen Reagens, dadurch gekennzeichnet, daß man das biologische oder biogene Material - gegebenenfalls nach Verdünnung mit pyrogenfreiem, sterilem Wasser - auf einen pH-Wert von etwa 6,5 einstellt und etwa 1 g/l eines Detergens, insbesondere eines anionischen Detergens, zusetzt und danach dieses Material unter Rühren langsam auf etwa 70°C erhitzt und bei dieser Temperatur mindestens 15, vorzugsweise etwa 30 min weiter rührt, wonach man es rasch abkühlt, auf einen pH von 4 bis 4, 2 ansäuert und die ausgefällten Globuline abtrennt, und daß man es nach der Detergens-/Warmebehandlung mit einem chaotropen Reagens mindestens 12, vorzugsweise 18 Stunden behandelt.

2. Verfahren nach Anspruch 1, dadurch gekennzeichnet, daß als chaotropes Reagens Harnstoff in einer Konzentration von 6-8 molar, bezogen auf die Gesamtemenge, eingesetzt wird.

3. Verfahren nach Anspruch 1, dadurch gekennzeichnet, daß als chaotropes Reagens Natriumthiocyanat in einer Konzentration von 1-2 molar, bezogen auf die Gesamtemenge, eingesetzt wird.

4. Verfahren nach einem der Ansprüche 1 bis 3, dadurch gekennzeichnet, daß das chaotrope Reagens nach der Behandlung durch Dialyse, Diafiltration oder Gelpermeationschromatographie entfernt wird.

5. Verfahren nach einem der Ansprüche 1-4, dadurch gekennzeichnet, daß man als Detergens ein Alkylsulfat oder ein Derivat davon, vorzugsweise Natriumlaurylsulfat, und/oder ein Sarcosinat, vorzugsweise Natrium-(N-Lauroylsarcosinat) und/oder ein Alkylsulfonat oder ein Derivat davon einsetzt.

6. Verfahren nach einem der Ansprüche 1 bis 5, dadurch gekennzeichnet, daß man den pH-Wert durch Zugabe verdünnter Salzsäure einstellt.

7. Verfahren nach einem der Ansprüche 1 bis 6, dadurch-gekennzeichnet, daß man zusätzlich zu dem Detergens 8-10 Vol.-% Methyl- oder Ethylalkohol zusetzt.

## Claims

1. Process for deactivating prions and viruses in biological and biogenic materials, in particular in a plasma or serum suitable as starting material for albumin, using a chaotropic reagent, characterized in that the biological or biogenic material is adjusted to a pH of about 6.5 - optionally after dilution with pyrogen-free, sterile water -, and about 1 g/l of a detergent, in particular of an anionic detergent, is added, in that thereafter this material is slowly heated to about 70°C while stirring and is further stirred for at least 15, preferably about 30, minutes at this temperature, after which it is rapidly cooled and acidified to a pH of from 4 to 4.2 and the precipitated globulins are isolated, and in that this material is treated with a chaotropic reagent for at least 12, preferably 18, hours after the detergent/heat treatment.

2. Process according to claim 1, characterized in that urea in a 6-8 molar concentration, based on the total amount, is used as the chaotropic reagent.

3. Process according to claim 1, characterized in that sodium thiocyanate in a 1-2 molar concentration, based on the total amount, is used as the chaotropic reagent.

4. Process according to any of claims 1 to 3, characterized in that the chaotropic reagent is removed by dialysis, dia-filtration or gel permeation chromatography after the treatment.

5. Process according to any of claims 1 to 4, characterized in that an alkylsulphate or a derivative thereof, preferably sodium laurylsulphate, and/or a sarcosinate, preferably sodium N-lauroylsarcosinate, and/or an alkane-sulphonate or a derivative thereof are used as the detergent.

6. Process according to any of claims 1 to 5, characterized in that the pH is adjusted by adding dilute hydrochloric acid.

7. Process according to any of Claims 1 to 6, characterized in that 8-10% by volume of methyl or ethyl alcohol are added in addition to the detergent.

## Revendications

1. Une méthode d'inactivation de prions et de virus dans des substances biologiques et biogénétiques, en particulier dans un plasma ou un sérum convenant comme matière première pour l'obtention d'albumine, avec utilisation d'un réactif chaotropique, caractérisée en ce qu'on ajuste - éventuellement après dilution avec de l'eau exempte de substances pyrogènes stérile-le pH à 6,5 environ et ajoute environ 1 g/l de détergent, en particulier de détergent anionique, aux substances biologiques ou biogénétiques, en ce qu'ensuite on chauffe lentement cette substance à environ 70°C sous agitation et poursuit l'agitation à cette température pendant au moins 15, de préférence 30 minutes, puis, on refroidit brutalement, acidifie à un pH de 4 à 4,2 et sépare la globuline précipitée, et en ce qu'on traite cette substance - après le traitement avec détergent/chaude - à l'aide de réactif chaotropique pendant au moins 12, de préférence 18 heures.

2. Méthode selon la revendication 1, caractérisée en ce que, en tant que réactif chaotropique, on utilise de l'urée a une concentration de 6-8 molaire, exprimée par rapport à la quantité totale.

3. Méthode selon la revendication 1, caractérisée en ce que, en tant que réactif chaotropique, on utilise du thiocyanate de sodium à une concentration de 1-2 molaire, par rapport à la quantité totale.

4. Méthode selon l'une des revendications 1 à 3, caractérisée en ce que le réactif chaotropique est éliminé, après le traitement, par dialyse, diafiltration ou chromatographie par perméation de gel.

5. Méthode selon l'une des revendications 1 à 4, caractérisée en ce que, en tant que détergent, on utilise un sulfate d'alkyle ou un de ses dérivés, de préférence du laurylsulfate de sodium, et/ou un sarcosinate, de préférence, le N-laurylsarcosinate de sodium, et/ou un alkyl sulfonate ou un de ses dérivés.

6. Méthode selon l'une des revendications 1 à 5, caractérisée en ce qu'on ajuste le pH par addition d'acide chlorhydrique dilué.

7. Méthode selon l'une des revendications 1 à 6, caractérisée en ce qu'en plus du détergent, on ajoute 8-10% en vol. d'alcool méthylique ou éthylique.
